# EUROPEAN PATENT SPECIFICATION

(11) **EP 4 572 841 B1**
(45) Date of publication and mention of the grant of the patent: **02.12.2026**
(21) Application number: 23794113.3
(22) Date of filing: 13.10.2023
(51) Int. Cl.: A61N 1/36, A61N 1/06, A61N 1/40, G16H 30/40, G16H 50/50

(54) **APPARATUS FOR SMALL ARRAY DOSE DISTRIBUTION OF ALTERNATING ELECTRIC FIELDS**
VORRICHTUNG ZUR VERTEILUNG DER DOSIS VON ELEKTRISCHEN WECHSELFELDERN MIT KLEINER ANORDNUNG
APPAREIL DE DISTRIBUTION DE PETITE DOSE EN RÉSEAU DE CHAMPS ÉLECTRIQUES ALTERNATIFS

(30) Priority: 14.10.2022 US 202263416408 P; 12.10.2023 US 202318379256
(43) Date of publication of application: 25.06.2025
(73) Proprietor: Novocure GmbH, 6340 Baar (CH)
(72) Inventor: NAVEH, Ariel, 31905 Haifa (IL)
(74) Representative: Carpmaels & Ransford LLP
(86) International application number: PCT/IB2023/060361
(87) International publication number: WO 2024/079716

(56) References cited:
- US-A1- 2021 060 334
- US-A1- 2021 299 439
- US-A1- 2022 096 829
- US-A1- 2022 096 853

## Description

### BACKGROUND

Tumor treating fields (TTFields) are low intensity alternating electric fields within the intermediate frequency range (for example, 50 kHz to 1 MHz), which may be used to treat tumors as described in U.S. Patent No. 7,565,205. TTFields are induced non-invasively into the region of interest by transducers placed on the patient's body and applying alternating current (AC) voltages between the transducers. Conventionally, a first pair of transducers and a second pair of transducers are placed on the subject's body. AC voltage is applied between the first pair of transducers for a first interval of time to generate an electric field with field lines generally running in the front-back direction. Then, AC voltage is applied at the same frequency between the second pair of transducers for a second interval of time to generate an electric field with field lines generally running in the right-left direction. The system then repeats this two-step sequence throughout the treatment.

US-A-2022/0096829 discloses a computer-implemented method to determine placement of transducers on a subject's body for inducing tumor treating fields, and a method and apparatus for applying tumor treating fields to a torso of a subject's body.

US-A-2021/0299439 discloses methods, systems and apparatuses for assisting transducer placements on a subject's body for applying tumor treating fields.

US-A-2022/0096853 discloses methods and systems for determining placement positions for one or more transducer arrays on a subject's body.

US-A-2021/0060334 discloses a method for planning the positioning of sets of electrode elements on a subject's body, and a method for delivering tumor treating fields to the neck of a subject.

### SUMMARY OF THE INVENTION

In one aspect the present invention provides a computer-implemented method to determine locations of transducers to apply tumor treating fields to a target tissue of a subject's body according to claim 1.

In another aspect the present invention provides a system to apply tumor treating fields to a target tissue of a subject's body according to claim 9.

Embodiments of the invention are disclosed in the dependent claims.

### BRIEF DESCRIPTION OF THE DRAWINGS

FIG. 1 is a flowchart depicting an example computer-implemented method of determining locations of transducers to apply TTFields to a target tissue of a subject's body.
Fig. 2 is a flowchart depicting an example method for applying TTFields to a subject's body.
FIGS. 3A-3I depict example transducer layouts at various locations for applying TTFields to a subject's body.
FIGS. 4A-4I depict example mean field intensities for the various transducer layouts of FIG. 3.
FIG. 5 depicts the variability of various transducer layouts showing model resistance vs. relative array area.
FIG. 6 depicts an example apparatus to apply alternating electric fields to a subject's body.
FIGS. 7A-7B illustrate schematic views of exemplary design of a transducer for applying alternating electric fields.
FIG. 8 depicts an example computer apparatus.

### DESCRIPTION OF EMBODIMENTS

Embodiments described hereinafter are only of exemplary and illustrative nature and provided for better understanding the invention which is defined by the appended claims.

This application describes exemplary methods, apparatuses, and systems to determine locations of transducers to apply alternating electric fields (e.g., tumor treating fields (TTFields)) to a target tissue of a subject's body and to apply an alternating electric field to a subject's body.

In preparing to receive TTFields therapy, a subject or a caregiver may have difficulty placing a transducer on a subject at a desired position due to, for example, a chemo port on the subject, a sensitive scar area of the subject, an anatomic part of the subject (e.g., an ear or a nipple), and/or even desired clothing for the subject to wear. From receiving TTFields therapy, a subject may experience skin sensitivity at the location of the transducer. In considering these problems, the inventor discovered that a smaller transducer (i.e., a transducer having a smaller area in contact with the subject) may help to alleviate at least some of these problems. For example, with a smaller transducer, the transducer may be placed on the subject to avoid a chemo port on the subject, a sensitive scar area of the subject, and/or an anatomic part of the subject (e.g., an ear or a nipple). Further, with a smaller transducer, the subject may have more clothing options which also conceal the transducer from public view. In addition, with a smaller transducer, less of the subject's skin may experience skin sensitivity at the location of the transducer. The inventor has further discovered that using a smaller transducer surprisingly may have increased effectiveness for TTFields. The smaller size of the transducer can be with respect to, for example, the size of another transducer and/or the size of the organ (or similar tissue type) (e.g., a lung) being treated with TTFields.

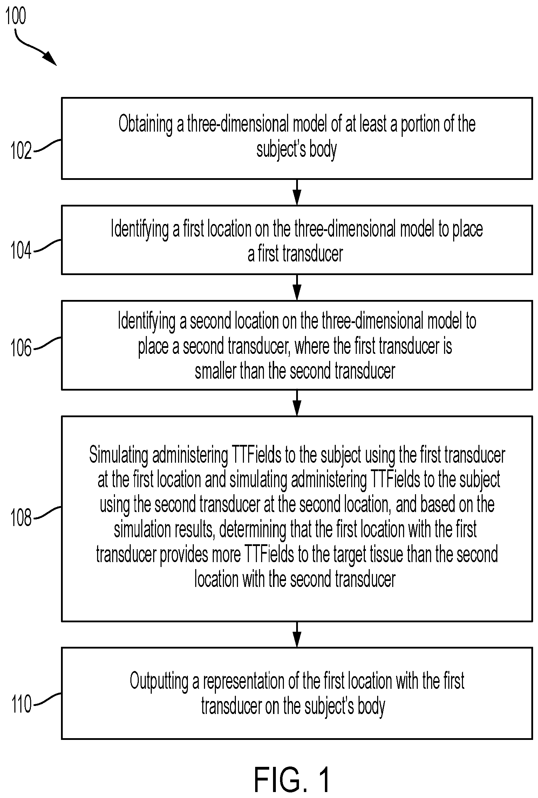

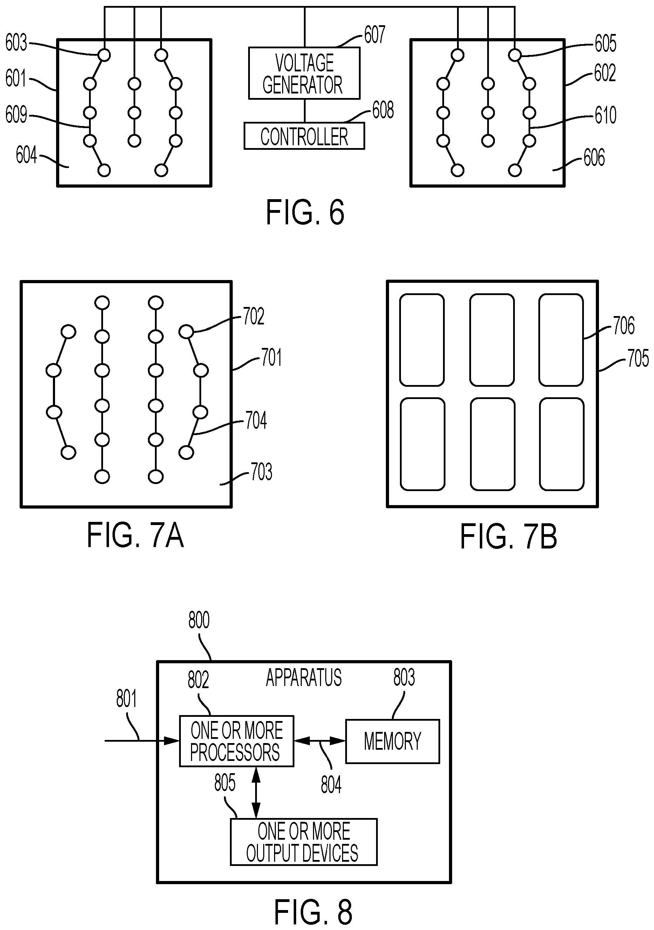

FIG. 1 is a flowchart depicting an example computer-implemented method 100 of determining locations of transducers to apply TTFields to a target tissue of a subject's body. In method 100, the impact of using different sized transducers to administer TTFields is determined via computer simulation. The computer may be, for example, any device comprising one or more processors and memory accessible by the one or more processors, the memory storing instructions that when executed by the one or more processors cause the computer to perform the relevant steps of the method 100. The method 100 may be implemented by any suitable system or apparatus, such the apparatus of FIG. 8. While an order of operations is indicated in FIG. 1 for illustrative purposes, the timing and ordering of such operations may vary where appropriate without negating the purpose and advantages of the examples set forth in detail herein.

With reference to FIG. 1, at step 102, the method 100 includes obtaining a three-dimensional (3D) model of at least a portion of the subject's body. The three-dimensional model may be obtained from computer memory (or computer storage) locally or over a network. The three-dimensional model may be generated based on one or more images of a region of the subject. In some embodiments, the one or more images are medical images. The medical image may, for example, include at least one of a magnetic resonance imaging (MRI) image, a computerized tomography (CT) image, an X-ray image, an ultrasound image, nuclear medicine image, a positron-emission tomography (PET) image, arthrogram images, myelogram images, or any image of the subject's body providing an internal view of the subject's body. Each medical image may include an outer shape of a portion of the subject's body and a region corresponding to a region of interest (e.g., tumor) within the subject's body. As an example, the medical image may be a 3D MRI image.

At step 104, the method 100 includes identifying a first location on the three-dimensional model to place a first transducer. The first location may be any location on a subject's body. For example, the first location may be on the torso of the subject or on the head of the subject.

At step 106, the method 100 includes identifying a second location on the three-dimensional model to place a second transducer. Similar to the first location, the second location may be any location on a subject's body. For example, the second location may be on the torso of the subject or on the head of the subject. However, since the impact of the first transducer and the second transducer on the application of TTFields are to be compared, the second location may be about, close to, nearby, or around the location of the first location. The second transducer and the second location of step 106 may be considered to be alternates to the first transducer and the first location of step 104. In some embodiments, the first transducer may be smaller than the second transducer. In some embodiments, the second transducer may be larger than the first transducer.

In some embodiments, the first transducer or the second transducer may be adapted to be located on a particular part of the subject (e.g., the torso of the subject or the head of the subject).

In some embodiments, the first transducer and the second transducer may have various shapes and/or sizes. For example, the first transducer or the second transducer may be triangular, rectangular, circular, oval, ovaloid, ovoid, or elliptical in shape or substantially triangular, substantially rectangular, substantially circular, substantially oval, substantially ovaloid, substantially ovoid, or substantially elliptical in shape.

In some embodiments, the first and second transducer each may have at least one electrode element adapted to provide TTFields. As an example, at least one electrode element of the first transducer or the second transducer may include at least one ceramic disk (e.g., diameter ranging from approximately 2 cm to approximately 3 cm) that is adapted to generate an alternating electric field. As an example, at least one electrode element of the first transducer or the second transducer may include a polymer film that is adapted to generate an alternating electric field.

At step 108, the method 100 includes simulating administering TTFields to the subject using the first transducer at the first location and simulating administering TTFields to the subject using the second transducer at the second location, and based on the simulation results, determining whether the first location with the first transducer or the second location with the second transducer provides more TTFields to the target tissue. If the first transducer is smaller than the second transducer, the comparison of the simulation results may determine that the first location with the first transducer provides more TTFields to the target tissue than the second location with the second transducer.

In some embodiments, the transducers may have various sizes such that a size of the first transducer is smaller than a size of the second transducer. The sizes of the first transducer and the second transducer may be compared by viewing the transducer from a direction perpendicular to a first surface of the transducer, where the first surface of the transducer is the surface of the transducer facing the subject and/or for attaching the transducer to the subject. The size of the first transducer and the second transducer may be determined by viewing the transducer from a direction perpendicular to the first surface of the transducer and calculating an area of the electrode elements of the transducer. The area of the electrode elements of the transducer may be used as the area of the transducer since the area of the electrode elements is the active area of the transducer adapted to provide the electric field to the subject. As an example, when viewed from a direction perpendicular to the first surface of the transducer, if the transducer has one electrode element with an area of 25 cm², the area of the transducer is 25 cm². As an example, when viewed from a direction perpendicular to the first surface of the transducer, if the transducer has four electrode elements with an area of 25 cm² each, the area of the transducer is 100 cm². As such, when viewed from a direction perpendicular to the first surface of the transducer, the size of the transducer may be referred to as an area of the at least one electrode element of the transducer.

As an example, when viewed from a direction perpendicular to the first surface of the first transducer and when viewed from a direction perpendicular to the first surface of the second transducer, an area of the at least one electrode element of the first transducer is less than or equal to approximately 50% of an area of the at least one electrode element of the second transducer. As an example, when viewed from a direction perpendicular to the first surface of the second transducer, an area of the at least one electrode element of the second transducer may range from approximately 300 cm² to approximately 525 cm², and when viewed from a direction perpendicular to the first surface of the first transducer, an area of at least one electrode element of the first transducer may range from approximately 150 cm² to approximately 262.5 cm² (or from approximately 150 cm² to approximately 265 cm²). As an example, an area of the at least one electrode element of the second transducer may range from approximately 300 cm² to approximately 400 cm², and an area of the at least one electrode element of the first transducer may range from approximately 150 cm² to approximately 200 cm². As an example, an area of the at least one electrode element of the second transducer may range from approximately 425 cm² to approximately 525 cm², and an area of the at least one electrode element of the first transducer may range from approximately 212.5 cm² to approximately 262.5 cm². As an example, an area of the at least one electrode element of the second transducer may be approximately 352 cm², and an area of the at least one electrode element of the first transducer may be approximately 176 cm². As an example, an area of the at least one electrode element of the second transducer may be approximately 475 cm², and an area of the at least one electrode element of the first transducer may be approximately 237.5 cm².

As an example, when viewed from a direction perpendicular to the first surface of the first transducer and when viewed from a direction perpendicular to the first surface of the second transducer, an area of the at least one electrode element of the first transducer is less than or equal to approximately 70% of an area of the at least one electrode element of the second transducer. As an example, an area of the at least one electrode element of the second transducer may be approximately 352 cm², and an area of the at least one electrode element of the first transducer may be approximately 246.4 cm².

At step 110, since the first location with the first transducer was determined in step 108 to provide more TTFields to the target tissue than the second location with the second transducer, the method 100 includes outputting a representation of the first location with the first transducer on the subject's body. In some embodiments, one or more recommended transducer placement positions may be generated based on, for example, the region of interest of the subject's body corresponding to the target tissue (e.g., a tumor in a single lung). As an example, the one or more recommended transducer placement positions may be intended to optimize the tumor treatment dose delivered to the region of interest of the subject's body. As an example, a display is used to show a representation of the first transducer and/or the first location on the subject's body. As an example, a display is used to show a representation of two pairs of transducers on the subject's body for delivering TTFields, where the first transducer is included as one of the transducers in the two pairs of transducers. As an example, a display is used to show a representation of two pairs of locations on the subject's body to place transducers for delivering TTFields, where the first location is included as one of the locations in the two pairs of locations. As an example, a document is used to show a representation of the two pairs of transducers and/or the two pairs of locations on the subject's body.

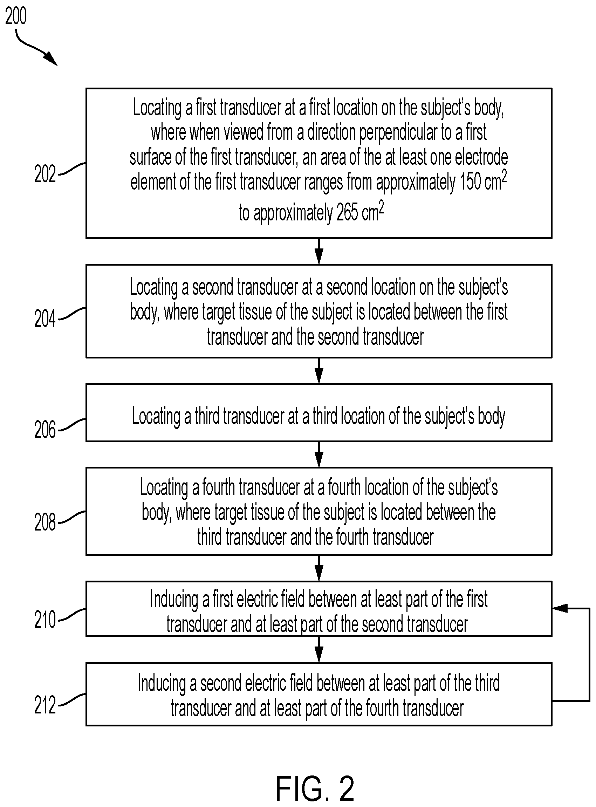

FIG. 2 is a flowchart depicting an example method 200 for applying TTfields to a subject's body. Using a smaller transducer selected as discussed with respect to FIG. 1, TFFields may be administered to a subject using the method 200. Certain steps of the method 200 are described as computer-implemented steps. The computer may be, for example, any device comprising one or more processors and memory accessible by the one or more processors, the memory storing instructions that when executed by the one or more processors cause the computer to perform the relevant steps of the method 200. The method 200 may be implemented by any suitable system or apparatus, such the apparatus of FIG. 8. While an order of operations is indicated in FIG. 2 for illustrative purposes, the timing and ordering of such operations may vary where appropriate without negating the purpose and advantages of the examples set forth in detail herein.

With reference to FIG. 2 at step 202, the method 200 includes locating a first transducer at a first location of the subject's body. The first transducer and the first location in step 202 may be the first transducer and the first location selected in method 100. The first transducer may be a smaller transducer. As an example, when viewed from a direction perpendicular to the first surface of the first transducer, an area of at least one electrode element of the first transducer may range from approximately 150 cm² to approximately 262.5 cm² (or from approximately 150 cm² to approximately 265 cm²). In some embodiments, the first transducer may have at least one electrode clement adapted to be coupled to a voltage generator.

At step 204, the method 200 includes locating a second transducer at a second location of the subject's body to pair with the first transducer at the first location of step 202 for administering TTFields to the subject. The second transducer at the second location of step 204 is not the same as the second transducer at a second location of step 106. The second transducer of step 204 may be a larger transducer and/or may be larger than the first transducer of step 202. As an example, when viewed from a direction perpendicular to the first surface of the second transducer, an area of at least one electrode element of the second transducer ranges from approximately 150 cm² to approximately 262.5 cm² (or from approximately 150 cm² to approximately 265 cm²). As an example, target tissue may be located between the first transducer and the second transducer. For example, the target tissue may be a tumor or cancer in a lung, which may be placed between the first and the second locations. In some embodiments, the second transducer may have at least one electrode element adapted to be coupled to the voltage generator.

At step 206, the method 200 includes locating a third transducer at a third location of the subject's body. At step 208, the method 200 includes locating a fourth transducer at a fourth location of the subject's body to pair with the third transducer at the third location of step 206 for administering TTFields to the subject. In some embodiments, the target tissue of the subject may be located between the third transducer and the fourth transducer. In some embodiments, the location of the third or fourth transducer may be overlapped with the first or second location. In some embodiments, the location of the third or fourth transducer may not be overlapped with the first or second location. As an example, all transducers (e.g., first transducer, second transducer, third transducer, and fourth transducer) may target the same tissue for additional or synergistic therapeutic effects of the alternating fields.

At step 210, the method 200includes inducing a first electric field between at least part of the first transducer and at least part of the second transducer by applying an AC voltage between this first pair of transducers. At step 212, the method 200 includes inducing a second electric field between at least part of the third transducer and at least part of the fourth transducer by applying an AC voltage between this first pair of transducers. Flow cycles between steps 210 and 212 to generate alternating electric fields at a particular interval for a particular period of time depending on a determined TTFields dosage.

As an example, an alternating electric field (e.g., TTFields) may be applied to target tissue (e.g., tumor or cancer in a lung), cells, or the area of a subject. In some embodiments, the alternating electric field may be applied with predetermined parameters. As an example, the alternating electric field may include a frequency within a frequency range from about 50 kHz to about 1 MHz. As an example, the alternating electric field may include a frequency within a frequency range from about 50 kHz to about 10,000 kHz. As an example, the frequency of the alternating electric field may be between approximately 50 kHz and approximately 1000 kHz or between approximately 100 kHz and approximately 300 kHz. As an example, the frequency of the alternating electric field may be approximately 100 kHz, approximately 150 kHz, approximately 200 kHz, approximately 250 kHz, or approximately 300 kHz.

As an example, the alternating electric fields (e.g., TTFields) may include an intensity within a range from about 1 V/cm to about 10 V/cm. As an example, the intensity of the alternating electric fields may be between approximately 1 V/cm and approximately 4 V/cm. Other possible exemplary parameters for the alternating electric field may include active time, dimming time, and duty cycle (all of which may be measured in, for example, ms units), among other parameters. The parameters may be modified based on the conditions of the subject (e.g., the sizes of the target tissue, type of tumor, age, or sex of the subject) or the purposes of the treatment. As an example, the intensity of the alternating electric field may be between approximately 1 V/cm and approximately 4 V/cm, and the frequency of the alternating electric field may be between approximately 150 kHz and approximately 250 kHz for treating tumor/cancer cells. In some embodiments, the alternating electric field may be applied using two pairs of transducer arrays placed on the subject and directed on a target tissue (e.g., tumor) of the subject.

In some embodiments, the portion of the subject's body to be treated with TTFields includes the target tissue. The target tissue may include cancer, tumor, lung, brain, or combinations thereof. In some embodiments, the target tissue may be located in an organ of the subject's body. As an example, cancer or tumor tissue in a lung of the subject's body may be the target tissue. In some embodiments, an area of the organ may be determined when viewed from a direction perpendicular to the first location for the first transducer. As an example, the area of the first transducer or the second transducer may be less than or equal to approximately 70% of the area of the organ. In some embodiments, the area of the first transducer may be less than or equal to approximately 50% of the area of the organ. In some embodiments, the organ may include a lung, a brain, a heart, or any tissue in a subject's body. For example, the target tissue may be cancer or tumor, and the organ may be the lung of the subject.

Various combinations of pairs of transducers, as discussed herein, or similar pairs of transducers may be used together. Various locations of transducers, such as those discussed herein or in other locations, may be used. A transducer may be used in a single pair of transducers or in two or more pairs of transducers. A transducer may be partitioned to be used in a single pair of transducers or in two or more pairs of transducers. The transducers, the transducer locations, the pairs of transducers, and the two or more pairs of transducers discussed herein are not exhaustive.

### EXPERIMENTAL RESULTS

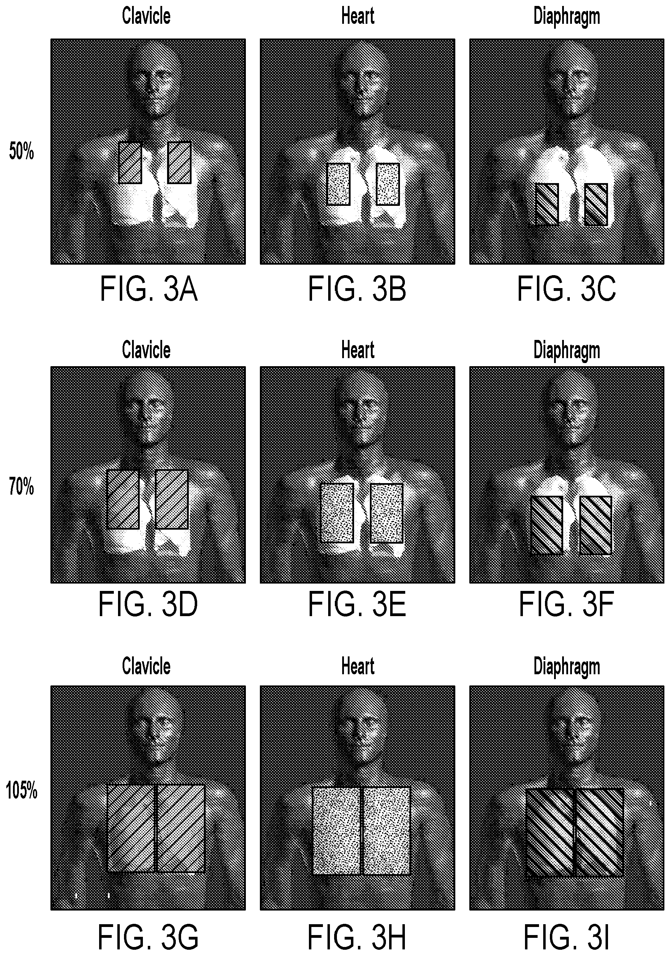

FIGS. 3A-3I depict example transducer layouts at various locations for applying TTFields to a subject's body. Transducer layouts of different sizes were placed at different heights relative to the lungs. In particular, a different size (e.g., 50%, 70%, and 105%) of the width and length of a typical transducer used for delivering TTFields to the lungs was simulated. As depicted in FIGS. 3A-3C, transducers that are 50% of the width and length of a typical transducer were simulated. As depicted in FIGS. 3D-3F, transducers that are 70% of the width and length of a typical transducer were simulated. As depicted in FIGS. 3G-3I, transducers that are 105% of the width and length of a typical transducer were simulated. As an example, a typical transducer may have an area of the at least one electrode element ranging from approximately 300 cm² to approximately 525 cm².

FIGS. 3A, 3D, and 3G show example transducers placed up to the clavicle bone. FIGS. 3B, 3E, and 3H show example transducers centrally aligned with the heart. FIGS. 3C, 3F, and 3I show the location of transducers where the bottom edge of the transducers coincides with the diaphragm.

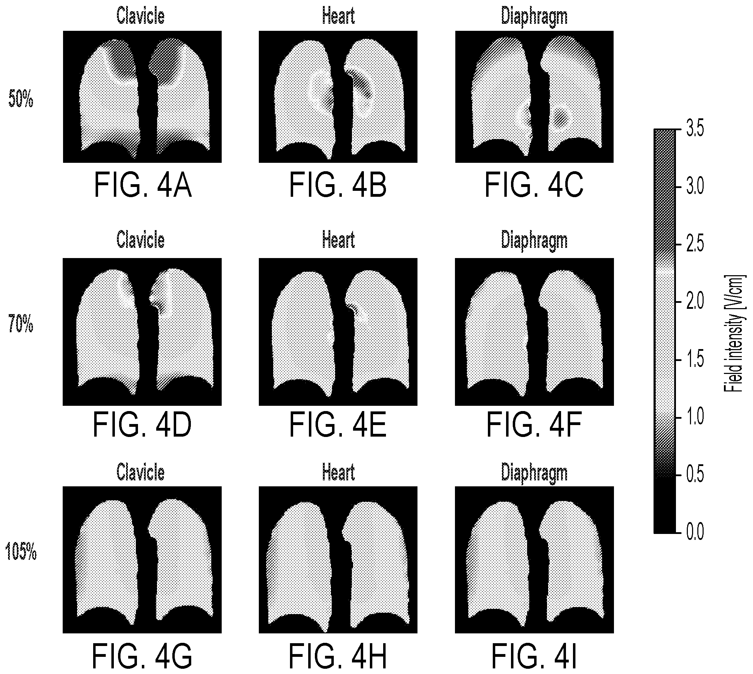

FIGS. 4A-4I depict example mean field intensities for the various transducer layouts of FIGS. 3A-3I, respectively. Using the transducer locations in FIGS. 3A-3I, the administration of TTFields was simulated using a computer system. As shown by FIGS. 4A, 4B, and 4C, when the transducer layout with 50% size of a typical transducer was used, the maximal values of the electric field intensity were directly related to the location of the transducers. Similarly, as shown by FIGS. 4D, 4E, and 4F, when the transducer layout with 75% size of a typical transducer was used, the area where the transducer was located (e.g., clavicle, heart, or diaphragm), showed the maximum mean field intensities. However, as shown by FIGS. 4G, 4H, and 4I, this effect diminishes as the transducers become larger. As discovered by the inventor, although the maximum value of the electric field intensity was significantly affected by the size and location of the transducer, the mean electric field intensity of the entire lung was similar regardless of the transducer's size or location.

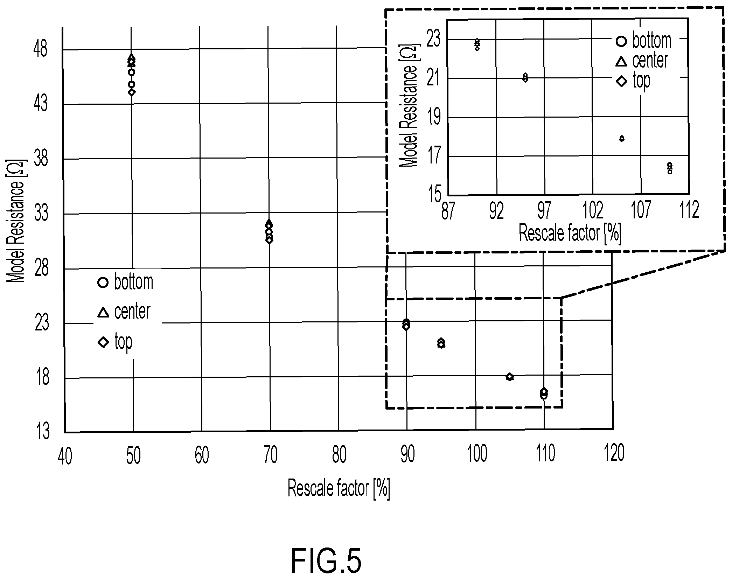

FIG. 5 depicts the variability of various transducer layouts showing model resistance vs. relative array area. FIG. 5 represents the values (i.e., model resistance vs. relative array area) for the three examined locations on the thorax (i.e., top, center, and bottom) such as those in FIGS. 3 and 4. In general, the variability of the resistance is shown to be strongly dependent on the size of the arrays rather than on their locations. For example, when the size of the transducer is smaller than 70% of the typical transducer, the variability of the resistance was larger compared to the size of the transducer, which is bigger than 90% of the typical transducer. As the transducers become larger, the variability of the resistance diminishes. The variance in resistance for the smallest arrays is about 5Ω, which is roughly 10% of the measured resistance.

### EXEMPLARY APPARATUSES

This application describes exemplary apparatuses to determine locations of transducers to apply alternating electric fields (e.g., TTFields) to a target tissue of a subject's body and to apply alternating electric fields to a subject's body.

In some embodiments, the system to apply TTFields to a target tissue of a subject's body may include a first transducer adapted to be located at a first location of the subject's body, a second transducer adapted to be located at a second location of the subject's body, a voltage generator adapted to provide a first voltage to the first transducer and a second voltage to the second transducer, and a controller coupled to the voltage generator.

FIG. 6 depicts an example apparatus to apply alternating electric fields (e.g., TTFields) to a subject's body. The first transducer array 601 includes 13 electrode elements 603, which are positioned on the substrate 604, and the electrode elements 603 are electrically and mechanically connected to one another through a conductive wiring 609. The second transducer array 602 includes 13 electrode elements 605, which are positioned on the substrate 606, and the electrode elements 605 are electrically and mechanically connected to one another through a conductive wiring 610. The first transducer array 601 and the second transducer array 602 are connected to an AC voltage generator 607 and a controller 608. The controller 608 may include one or more processors and memory accessible by the one or more processors. The memory may store instructions that when executed by the one or more processors, control the AC voltage generator 607 to implement one or more embodiments of the invention. In some embodiments, the AC voltage generator 607 and the controller 608 may be integrated in the first transducer array 601 and the second transducer array 602 and form a first electric field generator and a second electric field generator.

The structure of the transducers may take many forms. The transducers may be affixed to the subject's body or attached to or incorporated in clothing covering the subject's body. The transducer may include suitable materials for attaching the transducer to a subject's body. For example, the suitable materials may include cloth, foam, flexible plastic, and/or a conductive medical gel. The transducer may be conductive or nonconductive.

The transducer may include any desired number of electrode elements. Various shapes, sizes, and materials may be used for the electrode elements. Any constructions for implementing the transducer (or electric field generating device) for use with embodiments of the invention may be used as long as they are capable of (a) delivering TTFields to the subject's body and (b) being positioned at the locations specified herein. In some embodiments, at least one electrode element of the first, the second, the third, or the fourth transducer may include at least one ceramic disk that is adapted to generate an alternating electric field. In non-limiting embodiments, at least one electrode element of the first, the second, the third, or the fourth transducer may include a polymer film that is adapted to generate an alternating electric field. In some embodiments, the disclosed systems can have more than four transducers.

FIG. 7A illustrates a schematic view of an exemplary design of a transducer for applying alternating electric fields. The transducer array 701 includes 20 electrode elements 702, which are positioned on the substrate 703, and the electrode element 702 are electrically and mechanically connected to one another through a conductive wiring 704. In some embodiments, the electrode elements 702 may include a ceramic disk.

FIG. 7B illustrates a schematic view of an exemplary design of a transducer for applying alternating electric fields. The transducer 705 may include substantially flat electrode elements 706. In some embodiments, the electrode elements 706 are non-ceramic dielectric materials positioned over flat conductors. Examples of non-ceramic dielectric materials positioned over flat conductors may include polymer films disposed over pads on a printed circuit board or over flat pieces of metal. In some embodiments, such polymer films have a high dielectric constant, such as, for example, a dielectric constant greater than 10. In some embodiments, electrode elements 706 may have various shapes. For example, the electrode elements may be triangular, rectangular, circular, oval, ovaloid, ovoid, or elliptical in shape or substantially triangular, substantially rectangular, substantially circular, substantially oval, substantially ovaloid, substantially ovoid, or substantially elliptical in shape. In some embodiments, each of electrode elements 706 may have a same shape, similar shapes, and/or different shapes.

FIG. 8 depicts an example computer apparatus for use with the embodiments herein. As an example, the apparatus 800 may be a computer to implement certain inventive techniques disclosed herein. As an example, the apparatus 800 may be a controller apparatus to apply the alternating electric fields (e.g., TTFields) according to the embodiments herein. The controller apparatus 800 may be used as the controller 608 of FIG. 6. The apparatus 800 may include one or more processors 802, memory 803, one or more input devices, and one or more output devices 805.

In some embodiments, based on input 801, the one or more processors 802 may generate control signals to control the voltage generator to implement one or more embodiments descrived herein. As an example, the input 801 is user input. As an example, the input 801 may be from another computer in communication with the apparatus 800. The input 801 may be received in conjunction with one or more input devices (not shown) of the apparatus 800.

The memory 803 may be accessible by the one or more processors 802 (e.g., via a link 804) so that the one or more processors 802 can read information from and write information to the memory 803. The memory 803 may store instructions that when executed by the one or more processors 802 implement one or more embodiments described herein. The memory 803 may be a non-transitory computer readable medium (or a non-transitory processor readable medium) containing a set of instructions thereon for identifying and outputting a representation of a first location with a first transducer on a subject's body, where when executed by a processor (such as one or more processors 802), the instructions cause the processor to perform one or more methods disclosed herein.

The one or more output devices 805 may provide the status of the operation of the invention, such as transducer array selection, voltages being generated, and other operational information. The one or more output devices 805 may provide visualization data according to some embodiments described herein.

The apparatus 800 may be an apparatus including: one or more processors (such as one or more processors 802); and memory (such as memory 803) accessible by the one or more processors, the memory storing instructions that when executed by the one or more processors, cause the apparatus to perform one or more methods described herein.

Numerous modifications, alterations, and changes to the described embodiments are possible without departing from the scope of the present invention defined in the following claims.

## Claims

1. A computer-implemented method to determine locations of transducers (601, 602; 701; 705) to apply tumor treating fields to a target tissue of a subject's body, comprising:
obtaining a three-dimensional model of at least a portion of the subject's body; and
identifying a first location on the three-dimensional model to place a first transducer (601; 701; 705);
wherein the first transducer (601; 701; 705) has a first surface to be located facing the subject's body, the first transducer (601; 701; 705) has at least one electrode element (603; 702; 706) adapted to provide tumor treating fields, and, when viewed from a direction perpendicular to the first surface of the first transducer (601; 701; 705), an area of the at least one electrode element (603; 702; 706) of the first transducer (601; 701; 705) ranges from approximately 150 cm² to approximately 265 cm².

2. The method of claim 1, wherein at least one electrode element (603, 605; 702) of the first transducer (601; 701) comprises at least one ceramic disk that is adapted to generate an alternating electric field.

3. The method of claim 1, wherein at least one electrode element (706) of the first transducer (705) comprises a polymer film that is adapted to generate an alternating electric field.

4. The method of claim 1, wherein the first transducer (601; 701; 705) is triangular, rectangular, circular, oval, ovaloid, ovoid or elliptical in shape or substantially triangular, substantially rectangular, substantially circular, substantially oval, substantially ovaloid, substantially ovoid or substantially elliptical in shape.

5. The method of claim 1, wherein the target tissue is located in an organ of the subject's body, an area of the organ is determined when viewed from a direction perpendicular to the first location for the first transducer (601; 701; 705), and the area of the first transducer (601; 701; 705) is less than or equal to approximately 70% of the area of the organ.

6. The method of claim 5, wherein the area of the first transducer (601; 701; 705) is less than or equal to approximately 50% of the area of the organ.

7. The method of claim 1, wherein the first transducer (601; 701; 705) is adapted to be located on the torso.

8. The method of claim 1, further comprising:
identifying a second location on the three-dimensional model to place a second transducer (602; 701; 705);
wherein the second transducer (602; 701; 705) has a second surface to be located facing the subject's body, the second transducer (602; 701; 705) has at least one electrode element (605; 702; 706) adapted to provide tumor treating fields, and, when viewed from a direction perpendicular to the second surface of the second transducer (602; 701; 705), an area of the at least one electrode element (605; 702; 706) of the second transducer (602; 701; 705) ranges from approximately 150 cm² to approximately 265 cm².

9. A system to apply tumor treating fields to a target tissue of a subject's body, comprising:
a first transducer (601; 701; 705) adapted to be located at a first location of the subject's body;
a second transducer (602; 701; 705) adapted to be located at a second location of the subject's body;
wherein the target tissue is to be located between the first transducer (601; 701; 705) and the second transducer (602; 701; 705);
a voltage generator (607) adapted to provide a first voltage to the first transducer (601; 701; 705) and a second voltage to the second transducer (602; 701; 705); and
a controller (608) coupled to the voltage generator (607) and adapted to instruct the voltage generator (607) to induce a first alternating electric field between at least part of the first transducer (601; 701; 705) and at least part of the second transducer (602; 701; 705);
wherein the first transducer (601; 701; 705) has a first surface to be located facing the subject's body, the first transducer (601; 701; 705) has at least one electrode element (603; 702; 706) adapted to be coupled to the voltage generator (607), and, when viewed from a direction perpendicular to the first surface of the first transducer (601; 701; 705), an area of the at least one electrode element (603; 702; 706) of the first transducer (601; 701; 705) ranges from approximately 150 cm² to approximately 265 cm².

10. The system of claim 9, wherein the second transducer (602; 701; 705) has a second surface to be located facing the subject's body, the second transducer (602; 701; 705) has at least one electrode element (605; 702; 706) adapted to be coupled to the voltage generator (607), and, when viewed from a direction perpendicular to the second surface of the second transducer (602; 701; 705), an area of the at least one electrode element (605; 702; 706) of the second transducer (602; 701; 705) ranges from approximately 150 cm² to approximately 265 cm².

11. The system of claim 9, wherein at least one electrode element (603, 605; 702) of the first transducer (601; 701) or the second transducer (602; 701) comprises at least one ceramic disk that is adapted to generate an alternating electric field.

12. The system of claim 9, wherein at least one electrode element (706) of the first transducer (705) or the second transducer (705) comprises a polymer film that is adapted to generate an alternating electric field.

13. The system of claim 9, wherein the first transducer (601; 701; 705) or the second transducer (602; 701; 705) is triangular, rectangular, circular, oval, ovaloid, ovoid or elliptical in shape or substantially triangular, substantially rectangular, substantially circular, substantially oval, substantially ovaloid, substantially ovoid or substantially elliptical in shape.

14. The system of claim 9, further comprising:
a third transducer (701; 705) adapted to be located at a third location of the subject's body; and
a fourth transducer (701; 705) adapted to be located at a fourth location of the subject's body;
wherein the target tissue is to be located between the third transducer (701; 705) and the fourth transducer (701; 705);
wherein the voltage generator (607) is adapted to provide a third voltage to the third transducer (701; 705) and a fourth voltage to the fourth transducer (701; 705); and
wherein the controller (608) is adapted to instruct the voltage generator (607) to induce a second alternating electric field between at least part of the third transducer (701; 705) and at least part of the fourth transducer (701; 705).

## Patentansprüche

1. Computerimplementiertes Verfahren zum Bestimmen von Stellen von Wandlern (601, 602; 701; 705) zum Anwenden von Tumorbehandlungsfeldern auf ein Zielgewebe eines Körpers eines Patienten, umfassend:
Erhalten eines dreidimensionalen Modells von mindestens einem Abschnitt des Körpers des Patienten; und Identifizieren einer ersten Stelle auf dem dreidimensionalen Modell zum Platzieren eines ersten Wandlers (601; 701; 705);
wobei der erste Wandler (601; 701; 705) eine erste Oberfläche aufweist, die dem Körper des Patienten zugewandt ist, der erste Wandler (601; 701; 705) mindestens ein Elektrodenelement (603; 702; 706) aufweist, das dazu ausgelegt ist, Tumorbehandlungsfelder bereitzustellen, und eine Fläche des mindestens einen Elektrodenelements (603; 702; 706) des ersten Wandlers (601; 701; 705), betrachtet aus einer Richtung senkrecht zur ersten Oberfläche des ersten Wandlers (601; 701; 705), im Bereich von etwa 150 cm² bis etwa 265 cm² liegt.

2. Verfahren nach Anspruch 1, wobei mindestens ein Elektrodenelement (603, 605; 702) des ersten Wandlers (601; 701) mindestens eine Keramikscheibe umfasst, die dazu ausgelegt ist, ein elektrisches Wechselfeld zu erzeugen.

3. Verfahren nach Anspruch 1, wobei mindestens ein Elektrodenelement (706) des ersten Wandlers (705) einen Polymerfilm umfasst, der dazu ausgelegt ist, ein elektrisches Wechselfeld zu erzeugen.

4. Verfahren nach Anspruch 1, wobei der erste Wandler (601; 701; 705) dreieckig, rechteckig, kreisförmig, oval, ovaloid, eiförmig oder elliptisch oder im Wesentlichen dreieckig, im Wesentlichen rechteckig, im Wesentlichen kreisförmig, im Wesentlichen oval, im Wesentlichen ovaloid, im Wesentlichen eiförmig oder im Wesentlichen elliptisch ist.

5. Verfahren nach Anspruch 1, wobei sich das Zielgewebe in einem Organ des Körpers des Patienten befindet, eine Fläche des Organs, aus einer Richtung senkrecht zur ersten Stelle des ersten Wandlers (601; 701; 705) betrachtet, bestimmt wird und die Fläche des ersten Wandlers (601; 701; 705) kleiner oder gleich etwa 70 % der Fläche des Organs ist.

6. Verfahren nach Anspruch 5, wobei die Fläche des ersten Wandlers (601; 701; 705) kleiner oder gleich etwa 50 % der Fläche des Organs ist.

7. Verfahren nach Anspruch 1, wobei der erste Wandler (601; 701; 705) so ausgelegt ist, dass er sich am Oberkörper befindet.

8. Verfahren nach Anspruch 1, ferner umfassend:
Identifizieren einer zweiten Stelle auf dem dreidimensionalen Modell zum Platzieren eines zweiten Wandlers (602; 701; 705);
wobei der zweite Wandler (602; 701; 705) eine zweite Oberfläche aufweist, die dem Körper des Patienten zugewandt ist, wobei der zweite Wandler (602; 701; 705) mindestens ein Elektrodenelement (605; 702; 706) aufweist, das dazu ausgelegt ist, Tumorbehandlungsfelder bereitzustellen, und eine Fläche des mindestens einen Elektrodenelements (605; 702; 706) des zweiten Wandlers (602; 701; 705), betrachtet aus einer Richtung senkrecht zur zweiten Oberfläche des zweiten Wandlers (602; 701; 705), im Bereich von etwa 150 cm² und etwa 265 cm² liegt.

9. System zum Anwenden von Tumorbehandlungsfeldern auf ein Zielgewebe eines Körpers eines Patienten, umfassend:
einen ersten Wandler (601; 701; 705), der so ausgelegt ist, dass er sich an einer ersten Stelle des Körpers des Patienten befindet;
einen zweiten Wandler (602; 701; 705), der so ausgelegt ist, dass er sich an einer zweiten Stelle des Körpers des Patienten befindet;
wobei sich das Zielgewebe zwischen dem ersten Wandler (601; 701; 705) und dem zweiten Wandler (602; 701; 705) befinden soll;
einen Spannungsgenerator (607), der so ausgelegt ist, dass er dem ersten Wandler (601; 701; 705) eine erste Spannung und dem zweiten Wandler (602; 701; 705) eine zweite Spannung bereitstellt; und eine Steuereinheit (608), die mit dem Spannungsgenerator (607) gekoppelt ist und so ausgelegt ist, dass sie den Spannungsgenerator (607) anweist, ein erstes elektrisches Wechselfeld zwischen mindestens einem Teil des ersten Wandlers (601; 701; 705) und mindestens einem Teil des zweiten Wandlers (602; 701; 705) zu induzieren;
wobei der erste Wandler (601; 701; 705) eine erste Oberfläche aufweist, die dem Körper des Patienten zugewandt ist, der erste Wandler (601; 701; 705) mindestens ein Elektrodenelement (603; 702; 706) aufweist, das dazu ausgelegt ist, mit dem Spannungsgenerator (607) gekoppelt zu werden, und eine Fläche des mindestens einen Elektrodenelements (603; 702; 706) des ersten Wandlers (601; 701; 705), betrachtet aus einer Richtung senkrecht zur ersten Oberfläche des ersten Wandlers (601; 701; 705), im Bereich von etwa 150 cm² bis etwa 265 cm² liegt.

10. System nach Anspruch 9, wobei der zweite Wandler (602; 701; 705) eine zweite Oberfläche aufweist, die dem Körper des Patienten zugewandt ist, der zweite Wandler (602; 701; 705) mindestens ein Elektrodenelement (605; 702; 706) aufweist, das dazu ausgelegt ist, mit dem Spannungsgenerator (607) gekoppelt zu werden, und eine Fläche des mindestens einen Elektrodenelements (605; 702; 706) des zweiten Wandlers (602; 701; 705), betrachtet aus einer Richtung senkrecht zur zweiten Oberfläche des zweiten Wandlers (602; 701; 705), im Bereich von etwa 150 cm² bis etwa 265 cm² liegt.

11. System nach Anspruch 9, wobei mindestens ein Elektrodenelement (603, 605; 702) des ersten Wandlers (601; 701) oder des zweiten Wandlers (602; 701) mindestens eine Keramikscheibe umfasst, die dazu ausgelegt ist, ein elektrisches Wechselfeld zu erzeugen.

12. System nach Anspruch 9, wobei mindestens ein Elektrodenelement (706) des ersten Wandlers (705) oder des zweiten Wandlers (705) einen Polymerfilm umfasst, der dazu ausgelegt ist, ein elektrisches Wechselfeld zu erzeugen.

13. System nach Anspruch 9, wobei der erste Wandler (601; 701; 705) oder der zweite Wandler (602; 701; 705) dreieckig, rechteckig, kreisförmig, oval, ovaloid, eiförmig oder elliptisch oder im Wesentlichen dreieckig, im Wesentlichen rechteckig, im Wesentlichen kreisförmig, im Wesentlichen oval, im Wesentlichen ovaloid, im Wesentlichen eiförmig oder im Wesentlichen elliptisch ist.

14. System nach Anspruch 9, ferner umfassend:
einen dritten Wandler (701; 705), der so ausgelegt ist, dass er sich an einer dritten Stelle des Körpers des Patienten befindet; und
einen vierten Wandler (701; 705), der so ausgelegt ist, dass er sich an einer vierten Stelle des Körpers des Patienten befindet;
wobei sich das Zielgewebe zwischen dem dritten Wandler (701; 705) und dem vierten Wandler (701; 705) befinden soll;
wobei der Spannungsgenerator (607) so ausgelegt ist, dass er dem dritten Wandler (701; 705) eine dritte Spannung und dem vierten Wandler (701; 705) eine vierte Spannung bereitstellt; und wobei die Steuereinheit (608) so ausgelegt ist, dass sie den Spannungsgenerator (607) anweist, ein zweites elektrisches Wechselfeld zwischen mindestens einem Teil des dritten Wandlers (701; 705) und mindestens einem Teil des vierten Wandlers (701; 705) zu induzieren.

## Revendications

1. Procédé informatisé de détermination de l'emplacement de transducteurs (601, 602 ; 701 ; 705) pour appliquer des champs de traitement de tumeur à un tissu cible du corps d'un sujet, comprenant :
l'obtention d'un modèle tridimensionnel d'au moins une partie du corps du sujet ; et l'identification d'un premier emplacement sur le modèle tridimensionnel pour placer un premier transducteur (601 ; 701 ; 705) ;
dans lequel le premier transducteur (601 ; 701 ; 705) présente une première surface à placer face au corps du sujet, le premier transducteur (601 ; 701 ; 705) présente au moins un élément d'électrode (603 ; 702 ; 706) adapté pour fournir des champs de traitement de tumeur, et, lorsqu'il est observé d'une direction perpendiculaire à la première surface du premier transducteur (601 ; 701 ; 705), la surface de l'au moins un élément d'électrode (603 ; 702 ; 706) du premier transducteur (601 ; 701 ; 705) varie d'environ 150 cm² à environ 265 cm².

2. Procédé selon la revendication 1, dans lequel au moins un élément d'électrode (603, 605 ; 702) du premier transducteur (601 ; 701) comprend au moins un disque en céramique adapté pour générer un champ électrique alternatif.

3. Procédé selon la revendication 1, dans lequel au moins un élément d'électrode (706) du premier transducteur (705) comprend un film polymère adapté pour générer un champ électrique alternatif.

4. Procédé selon la revendication 1, dans lequel le premier transducteur (601 ; 701 ; 705) est de forme triangulaire, rectangulaire, circulaire, ovale, ovaloïde, ovoïde ou elliptique ou de forme sensiblement triangulaire, sensiblement rectangulaire, sensiblement circulaire, sensiblement ovale, sensiblement ovaloïde, sensiblement ovoïde ou sensiblement elliptique.

5. Procédé selon la revendication 1, dans lequel le tissu cible est situé dans un organe du corps du sujet, une zone de l'organe est déterminée lorsqu'elle est vue d'une direction perpendiculaire au premier emplacement du premier transducteur (601 ; 701 ; 705), et la zone du premier transducteur (601 ; 701 ; 705) est inférieure ou égale à environ 70 % de la zone de l'organe.

6. Procédé selon la revendication 5, dans lequel la surface du premier transducteur (601 ; 701 ; 705) est inférieure ou égale à environ 50 % de la surface de l'organe.

7. Procédé selon la revendication 1, dans lequel le premier transducteur (601 ; 701 ; 705) est adapté pour être situé sur le torse.

8. Procédé selon la revendication 1, comprenant en outre :
l'identification d'un deuxième emplacement sur le modèle tridimensionnel pour placer un deuxième transducteur (602 ; 701 ; 705) ;
dans lequel le deuxième transducteur (602 ; 701 ; 705) possède une deuxième surface à placer face au corps du sujet, le deuxième transducteur (602 ; 701 ; 705) possède au moins un élément d'électrode (605 ; 702 ; 706) adapté pour fournir des champs de traitement de tumeur, et, lorsqu'il est observé d'une direction perpendiculaire à la deuxième surface du deuxième transducteur (602 ; 701 ; 705), la surface de l'au moins un élément d'électrode (605 ; 702 ; 706) du deuxième transducteur (602 ; 701 ; 705) varie d'environ 150 cm² à environ 265 cm².

9. Système permettant d'appliquer des champs de traitement de tumeur à un tissu cible du corps d'un sujet, comprenant :
un premier transducteur (601 ; 701 ; 705) adapté pour être placé à un premier emplacement du corps du sujet ;
un deuxième transducteur (602 ; 701 ; 705) adapté pour être situé à un deuxième emplacement du corps du sujet ;
dans lequel le tissu cible doit être situé entre le premier transducteur (601 ; 701 ; 705) et le deuxième transducteur (602 ; 701 ; 705) ;
un générateur de tension (607) adapté pour fournir une première tension au premier transducteur (601 ; 701 ; 705) et une deuxième tension au deuxième transducteur (602 ; 701 ; 705) ; et
un dispositif de commande (608) couplé au générateur de tension (607) et adapté pour ordonner au générateur de tension (607) d'induire un premier champ électrique alternatif entre au moins une partie du premier transducteur (601 ; 701 ; 705) et au moins une partie du deuxième transducteur (602 ; 701 ; 705) ;
dans lequel le premier transducteur (601 ; 701 ; 705) présente une première surface à placer face au corps du sujet, le premier transducteur (601 ; 701 ; 705) présente au moins un élément d'électrode (603 ; 702 ; 706) adapté pour être couplé au générateur de tension (607), et, lorsqu'il est vu d'une direction perpendiculaire à la première surface du premier transducteur (601 ; 701 ; 705), la surface de l'au moins un élément d'électrode (603 ; 702 ; 706) du premier transducteur (601 ; 701 ; 705) varie d'environ 150 cm² à environ 265 cm².

10. Système selon la revendication 9, dans lequel le deuxième transducteur (602 ; 701 ; 705) possède une deuxième surface destinée à être orientée vers le corps du sujet, le deuxième transducteur (602 ; 701 ; 705) comporte au moins un élément d'électrode (605 ; 702 ; 706) adapté pour être couplé au générateur de tension (607), et, lorsqu'il est observé d'une direction perpendiculaire à la deuxième surface du deuxième transducteur (602 ; 701 ; 705), la surface de l'au moins un élément d'électrode (605 ; 702 ; 706) du deuxième transducteur (602 ; 701 ; 705) varie d'environ 150 cm² à environ 265 cm².

11. Système selon la revendication 9, dans lequel au moins un élément d'électrode (603, 605 ; 702) du premier transducteur (601 ; 701) ou du deuxième transducteur (602 ; 701) comprend au moins un disque en céramique adapté pour générer un champ électrique alternatif.

12. Système selon la revendication 9, dans lequel au moins un élément d'électrode (706) du premier transducteur (705) ou du deuxième transducteur (705) comprend un film polymère adapté pour générer un champ électrique alternatif.

13. Système selon la revendication 9, dans lequel le premier transducteur (601 ; 701 ; 705) ou le deuxième transducteur (602 ; 701 ; 705) est de forme triangulaire, rectangulaire, circulaire, ovale, ovaloïde, ovoïde ou elliptique ou de forme sensiblement triangulaire, sensiblement rectangulaire, sensiblement circulaire, sensiblement ovale, sensiblement ovaloïde, sensiblement ovoïde ou sensiblement elliptique.

14. Système selon la revendication 9, comprenant en outre :
un troisième transducteur (701 ; 705) adapté pour être placé à un troisième emplacement du corps du sujet ; et
un quatrième transducteur (701 ; 705) adapté pour être situé à un quatrième emplacement du corps du sujet ;
dans lequel le tissu cible doit être situé entre le troisième transducteur (701 ; 705) et le quatrième transducteur (701 ; 705) ;
dans lequel le générateur de tension (607) est adapté pour fournir une troisième tension au troisième transducteur (701 ; 705) et une quatrième tension au quatrième transducteur (701 ; 705) ; et dans lequel le dispositif de commande (608) est adapté pour ordonner au générateur de tension (607) d'induire un deuxième champ électrique alternatif entre au moins une partie du troisième transducteur (701 ; 705) et au moins une partie du quatrième transducteur (701 ; 705).
